# EUROPÄISCHE PATENTSCHRIFT

(11) **EP 2 155 390 B1**
(45) Veröffentlichungstag und Bekanntmachung des Hinweises auf die Patenterteilung: **17.10.2012**
(21) Anmeldenummer: 08749659.2
(22) Anmeldetag: 23.04.2008
(51) Int. Cl.: B01J 31/22, B01J 35/10, B01J 20/22, B01J 20/28, B01D 53/053, C07C 51/41, C07C 63/28

(54) **METALLORGANISCHE GERÜSTMATERIALIEN MIT HEXAGONAL-TRIGONALER STRUKTUR BASIEREND AUF ALUMINIUM, EISEN ODER CHROM, SOWIE EINER DICARBONSÄURE**
NEW ORGANOMETALLIC FRAMEWORK MATERIALS BASED ON ALUMINUM, IRON, AND CHROMIUM
NOUVEAUX MATÉRIAUX STRUCTURELS ORGANOMÉTALLIQUES À BASE D'ALUMINIUM, DE FER ET DE CHROME

(30) Priorität: 24.04.2007 EP 07106802
(43) Veröffentlichungstag der Anmeldung: 24.02.2010
(73) Patentinhaber: BASF SE, 67056 Ludwigshafen (DE)
(72) Erfinder: SCHUBERT, Markus, 67063 Ludwigshafen (DE); MÜLLER, Ulrich, 67435 Neustadt (DE); MARX, Stefan, 46049 Oberhausen (DE)
(86) Internationale Anmeldenummer: PCT/EP2008/054887
(87) Internationale Veröffentlichungsnummer: WO 2008/129051

(56) Entgegenhaltungen:
- WO-A-2007/023134
- WO-A-2007/101797
- US-A1- 2006 287 190
- RUSANOV E B ET AL: "A Topology Paradigm for Metal-Organic Zeolites" ANGEWANDTE CHEMIE INTERNATIONAL EDITION, Bd. 42, Nr. 22, 5. Juni 2003 (2003-06-05), Seiten 2499-2501, XP002529352
- MOULTON B ET AL: "Crystal Engineering of a Nanoscale Kagome Lattice" ANGEWANDTE CHEMIE INTERNATIONAL EDITION, Bd. 41, Nr. 15, 2. August 2002 (2002-08-02), Seiten 2821-2824, XP002529353
- BARTHELET K ET AL: "V(III)(OH){O2C-C6H4-CO2}.(HO2C-C6H4-CO2H) x(DMF)y(H2O)z (or MIL-68), a new vanadocarboxylate with a large pore hybrid topology: reticular synthesis with infinite inorganic building blocks?" CHEMICAL COMMUNICATIONS, Bd. 2004, Nr. 5, 5. Februar 2004 (2004-02-05), Seiten 520-521, XP002529354 in der Anmeldung erwähnt
- EDDAOUDI MOHAMED ET AL: "Geometric requirements and examples of important structures in the assembly of square building blocks" PROCEEDINGS OF THE NATIONAL ACADEMY OF SCIENCES OF USA, NATIONAL ACADEMY OF SCIENCE, WASHINGTON, DC.; US, Bd. 99, Nr. 8, 1. Januar 2002 (2002-01-01), Seiten 4900-4904, XP002476455 ISSN: 0027-8424
- JHUNG S H ET AL: "Microwave Synthesis of Chromium Terephthalate MIL-101 and Its Benzene Sorption Ability" ADVANCED MATERIALS, Bd. 19, Nr. 1, 7. Dezember 2006 (2006-12-07), Seiten 121-124, XP002529355
- LATROCHE M ET AL: "Hydrogen Storage in the Giant-Pore Metal-Organic Frameworks MIL-100 and MIL-101" ANGEWANDTE CHEMIE, Bd. 118, Nr. 48, 23. November 2006 (2006-11-23), Seiten 8407-8411, XP002529356
- FEREY G ET AL: "A Chromium Terephthalate?Based Solid with Unusually Large Pore Volumes and Surface Area" SCIENCE, Bd. 309, 23. September 2005 (2005-09-23), Seiten 2040-2042, XP002529357
- SONNAUER A ET AL: "Giant Pores in a Chromium 2,6-Naphthalenedicarboxylate Open- Framework Structure with MIL-101 Topology" ANGEWANDTE CHEMIE INTERNATIONAL EDITION, Bd. 48, Nr. 21, 4. Februar 2009 (2009-02-04), Seiten 3791-3794, XP002529358
- WHITFIELD TABATHA R ET AL: "Metal-organic frameworks based on iron oxide octahedral chains connected by benzenedicarboxylate dianions" SOLID STATE SCIENCES, ELSEVIER, PARIS, FR, Bd. 7, Nr. 9, 1. Januar 2005 (2005-01-01), Seiten 1096-1103, XP002476452 ISSN: 1293-2558

## Beschreibung

Die vorliegende Erfindung betrifft poröse metallorganische Gerüstmaterialien, Formkörper, diese enthaltend, sowie Verfahren zu deren Herstellung und Verwendung.

Poröse metallorganische Gerüstmaterialien sind im Stand der Technik bekannt. Sie zeichnen sich insbesondere durch ihre Porosität aus und sind häufig vergleichbaren Anwendung zuführbar, welche von anorganischen Zeolithen bekannt sind.

Metallorganische Gerüstmaterialien enthalten üblicherweise eine an ein Metallion koordinativ gebundene, mindestens zweizähnige organische Verbindung, die gemeinsam mit dem Metallion das Gerüst des metallorganischen Gerüstmaterials darstellt.

Die geeignete Wahl von Metall und/oder organischer Verbindung ermöglicht eine Optimierung für das gewünschte Anwendungsgebiet. Hierbei kann beispielsweise die Wahl der organischen Verbindung Einfluss auf die Porenverteilung nehmen. Darüber hinaus kann das Metall einen Beitrag bei Adsorptionsvorgehen liefern.

Darüber hinaus können jedoch Reaktionsbedingungen bei der Herstellung von metallorganischen Gerüstmaterialien und bei der Umsetzung verwendete Hilfsstoffe den Gerüstaufbau beeinflussen.

So werden beispielsweise zeolithähnliche metallorganische Gerüstmaterialien von Y. Liu et al., Chem. Commun. 14 (2006), 1488-1490, mit Hilfe von strukturdirigierenden Mitteln erzeugt. Weitere solcher Gerüstmaterialien sind in WO-A 2006/116340 beschrieben.

Es existiert also ein stetiger Bedarf, spezielle metallorganische Gerüstmaterialien bereitzustellen, die insbesondere außergewöhnliche Eigenschaften aufweisen, welche insbesondere auf spezielle Herstellverfahren zurückzuführen sind.

Als ein interessantes Metall kann Aluminium genannt werden, da aufgrund starker Koordinationsbindungen vergleichsweise robuste metallorganische Gerüstmaterialien erhalten werden können. Zudem ist das Al³⁺-Ion aufgrund seiner oktaedrischen Koordination prinzipiell geeignet, dreidimensionale Gerüstverbindungen aufzubauen. Weiterhin sind die als Einsatzstoffe verwendbaren Salze des Aluminiums gut zugänglich und preiswert. Aufgrund ihrer ähnlichen Struktur sind neben Aluminium-basierten metallorganischen Gerüstmaterialien auch diejenigen von Eisen und Chrom von Interesse.

Ein besonders interessantes metallorganisches Gerüstmaterial stellt Aluminiumterephthalat dar. Dieses wird von T. Loiseau et al., Chem. Eur. J. 10 (2004), 1373-1382, beschrieben. Durch konventionelle Herstellung ergibt sich eine Grundstruktur, bei der parallel angeordnete -Al-(OH)-Al-(OH)-Ketten in die zweite und dritte Dimension des Dicarbonlinkers überbrückt sind, so dass sie eindimensionale Kanäle mit einem rautenförmigen Querschnitt aufspannen. Die Grundstruktur ist in der Literatur als "MIL-53" bekannt. Das Kristallsystem ist (in der trockenen "ht"-Form) orthorombisch und die Raumgruppe lautet Imma. Die gleiche Struktur ergibt sich bei konventioneller Herstellung von Eisen- und Chromterephthalat (T.R. Whitfield et al., Solid State Sciences 7 (2005), 1096-1103; S. Bourrelly et al., J. Am. Chem. Soc. 127 (2005), 13519-13521).

Trotz der bekannten metallorganischen Gerüstmaterialien besteht ein Bedarf an alternativen Gerüstmaterialien, die überlegene Eigenschaften insbesondere in Bezug auf die Speicherung und Trennung von Gasen aufweisen.

RUSANOV E B ET AL: "A Topology Paradigm for Metal-Organic Zeolites" ANGEWANDTE CHEMIE INTERNATIONAL EDITION 42 (2003), 2499-2501 und MOULTON B ET AL: "Crystal Engineering of a Nanoscale Kagome Lattice "ANGEWANDTE CHEMIE INTERNATIONAL EDITION 41 (2002), 2821-2824 offenbaren Netzstrukturen in MOF-Materialien, welche in Anwesenheit von Hilfsstoffen sich zu einem Kagomé-Gitter in einem organischen Lösungsmittel (EtOH bzw. DMF) zusammenlagern können. Als Beispiele werden in letzterem Dokument Nitrobenzol, o-Dichlorbenzol und Naphthalin genannt.

WO 2007/101797 A, veröffentlicht am 13. September 2007, offenbart ein Verfahren zum Abtrennen von CO₂ mittels eines generisch definierten MOFs. Unter anderem ist eines der einsetzbaren MOFs MIL-68. Weiterhin ist Aluminium generell bevorzugt als Metall, wie auch die Kombination Al und Terephthalat.

Eine Aufgabe der vorliegenden Erfindung liegt somit in der Bereitstellung solcher Gerüstmaterialien sowie Verfahren zu deren Herstellung.

Die Aufgabe wird gelöst durch ein poröses metallorganisches Gerüstmaterial wie es in Anspruch 1 definiert ist.

Die Aufgabe wird ebenfalls gelöst durch ein Verfahren zur Herstellung eines erfindungsgemäßen porösen metallorganischen Gerüstmaterials gemäß Anspruch 4.

Es wurde nämlich gefunden, dass aufgrund des oben beschriebenen Herstellverfahrens unter Einsatz des definierten Hilfsstoffes neue metallorganische Gerüstmaterialien entstehen, die eine andere Struktur zu den konventionell hergestellten bekannten Strukturen aufweisen, die aus den gleichen Metallen und organischen Verbindungen aufgebaut sind.

Hierbei hat sich insbesondere in überraschender Weise ergeben, dass in Gegenwart des Hilfsstoffs eine Struktur ausgebildet wird, die als "MIL-68" für V^{III}-haltige metallorganische Gerüstmaterialien bekannt ist, wobei bei diesen Gerüstmaterialien die Struktur spontan erhalten wird. Vanadiumterephthalat ist von K. Barthelet et al., Chem. Commun. 2004, 520-521, beschrieben. Hierbei zeigt sich eine Struktur, deren Projektion entlang [001] ein Muster aufweist, bei dem jede Seite eines Sechsecks durch ein Dreieck begrenzt ist.

Die vorliegende Erfindung wird nachfolgend näher erläutert.
Fig. 1 zeigt skizzenhaft das oben beschriebene Muster, wobei die in Fig. 1 dargestellten Oktaeder die oktaedrisch koordinierten Metallzentren wiedergeben.
Fig. 2 zeigt das Röntgendiffraktogramm des erfindungsgemäßen Aluminiumterephthalats. Hierbei ist die Intensität I(Ln(Counts)) als Funktion der 2-Theta-Skala (2Θ) dargestellt.
Fig. 3 zeigt Adsorptionskurven für Kohlendioxid und Kohlenmonoxid an erfindungsgemäßem und aus dem Stand der Technik bekanntem Aluminiumterephthalat-Gerüstmaterial.

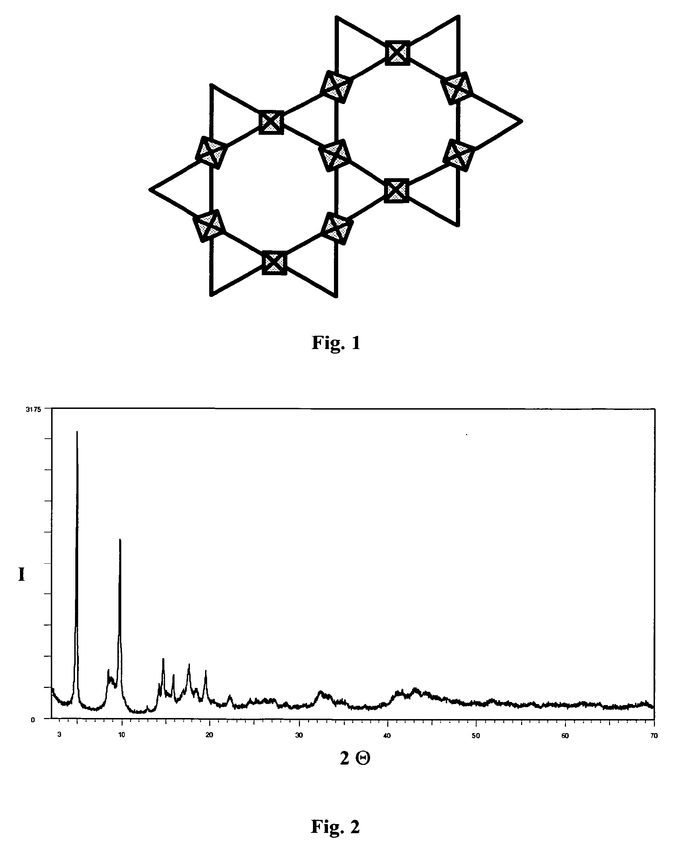

Fig. 1 zeigt das oben beschriebene Muster nur skizzenhaft. Eine detailliertere Darstellung ist in Figur 2 auf Seite 520 von K. Barthelet et al., Chem. Commun. 2004, 520-521, gezeigt.

Im Fall des Aluminiumterephthalats kann die Struktur des entsprechenden erfindungsgemäßen porösen metallorganischen Gerüstmaterials darin erkannt werden, dass das Röntgendiffraktogramm (XRD) einen Hauptreflex im Bereich von 4° < 2Θ < 6°, vorzugsweise im Bereich von 4,5° < 2Θ < 5,5° aufweist.

Hierbei kann das Diffraktogramm wie folgt ermittelt werden: Die Probe wird als Pulver in den Probenbehälter eines kommerziell erhältlichen Geräts (Siemens D-5000 Diffraktometer oder Bruker D8-Advance) eingebaut. Als Strahlungsquelle wird Cu-Kα-Strahlung mit variablen Primär- und Sekundärblenden und Sekundärmonochromator benutzt. Die Detektion des Signals erfolgt über einen Szintillations- (Siemens) oder Solex-Halbleiter Detektor (Bruker). Der Messbereich für 2θ wird typischerweise zwischen 2° und 70° gewählt. Der Winkelschritt beträgt 0,02°, die Messzeit pro Winkelschritt typischerweise 2 bis 4 Sekunden. Bei der Auswertung werden Reflexe durch eine wenigstens 3-fach höhere Signalstärke vom Grundrauschen unterschieden. Eine Flächenanalyse kann manuell erfolgen, indem an die einzelnen Reflexe eine Basislinie angelegt wird. Alternativ können Programme wie zum Beispiel "Topas-Profile" der Fa. Bruker eingesetzt werden, wobei die Untergrundanpassung dann bevorzugt über ein Polynom 1. Grades in der Software automatisch erfolgt.

Die Struktur des erfindungsgemäßen Gerüstmaterials Aluminiumterephthalat weist ein orthorhombisches Motiv auf und hat die Raumgruppe Cmcm.

Die verwendeten Abkürzungen für die Raumgruppen sind dem Fachmann geläufig und normiert. Hierbei sei beispielsweise auf die Internetseite der International Union of Crystallography (http://www.iucr.org/iucr-top/it/index.html) verwiesen.

Das erfindungsgemäße metallorganische Gerüstmaterial ist Aluminiumterephthalat.

Das erfindungsgemäße metallorganische Gerüstmaterial kann pulverförmig beziehungsweise als Agglomerat vorliegen.

Das erfindungsgemäße poröse metallorganische Gerüstmaterial kann als solches in Pulverform verwendet werden oder es wird in einen Formkörper umgewandelt.

Dementsprechend ist ein weiterer Aspekt der vorliegenden Erfindung, dass das erfindungsgemäße poröse metallorganische Gerüstmaterial als Pulver vorliegt.

Ein weiterer Aspekt der vorliegenden Erfindung ist demzufolge ein Formkörper enthaltend das erfindungsgemäße poröse metallorganische Gerüstmaterial.

Die Herstellung von Formkörpern aus metallorganischen Gerüstmaterialien ist beispielsweise in WO-A 03/102000 beschrieben.

Bevorzugte Verfahren zur Herstellung von Formkörpern sind hierbei die Verstrangung oder Tablettierung. Bei der Formkörperherstellung kann das Gerüstmaterial weitere Materialien, wie beispielsweise Binder, Gleitmittel oder andere Additive aufweisen, welche während der Herstellung hinzugesetzt werden. Ebenso ist es denkbar, dass das Gerüstmaterial weitere Bestandteile aufweist, wie zum Beispiel Absorbentien wie Aktivkohle oder dergleichen.

Hinsichtlich der möglichen Geometrien der Formkörper existieren im Wesentlichen keine Beschränkungen. Beispielsweise sind unter anderem Pellets wie beispielsweise scheibenförmige Pellets, Pillen, Kugeln, Granulat, Extrudate wie beispielsweise Stränge, Waben, Gitter oder Hohlkörper zu nennen.

Zur Herstellung dieser Formkörper sind grundsätzlich sämtliche geeigneten Verfahren möglich. Es sind insbesondere folgende Verfahrensführungen bevorzugt:
- Kneten/Kollern des Gerüstmaterials allein oder zusammen mit mindestens einem Bindemittel und/oder mindestens einem Anteigungsmittel und/oder mindestens einer Templatverbindung unter Erhalt eines Gemisches; Verformen des erhaltenen Gemisches mittels mindestens einer geeigneten Methode wie beispielsweise Extrudieren; Optional Waschen und/oder Trocknen und/oder Calcinieren des Extrudates; Optional Konfektionieren.
- Tablettieren zusammen mit mindestens einem Bindemittel und/oder anderem Hilfsstoff.
- Aufbringen des Gerüstmaterials auf mindestens ein gegebenenfalls poröses Trägermaterial. Das erhaltene Material kann dann gemäß der vorstehend beschriebenen Methode zu einem Formkörper weiterverarbeitet werden.
- Aufbringen des Gerüstmaterials auf mindestens ein gegebenenfalls poröses Substrat.

Kneten/Kollern und Verformen kann gemäß eines jeden geeigneten Verfahrens erfolgen, wie beispielsweise in Ullmanns Enzyklopädie der Technischen Chemie, 4. Auflage, Band 2, S. 313 ff. (1972) beschrieben.

Beispielsweise kann das Kneten/Kollern und/oder Verformen mittels einer Kolbenpresse, Walzenpresse in Anwesenheit oder Abwesenheit mindestens eines Bindermaterials, Compoundieren, Pelletieren, Tablettieren, Extrudieren, Co-Extrudieren, Verschäumen, Verspinnen, Beschichten, Granulieren, bevorzugt Sprühgranulieren, Versprühen, Sprühtrocknen oder einer Kombination aus zwei oder mehr dieser Methoden erfolgen.

Ganz besonders bevorzugt werden Pellets und/oder Tabletten hergestellt.

Das Kneten und/oder Verformen kann bei erhöhten Temperaturen wie beispielsweise im Bereich von Raumtemperatur bis 300 °C und/oder bei erhöhtem Druck wie beispielsweise im Bereich von Normaldruck bis hin zu einigen hundert bar und/oder in einer Schutzgasatmosphäre wie beispielsweise in Anwesenheit mindestens eines Edelgases, Stickstoff oder einem Gemisch aus zwei oder mehr davon erfolgen.

Das Kneten und/oder Verformen wird gemäß einer weiteren Ausführungsform unter Zugabe mindestens eines Bindemittels durchgeführt, wobei als Bindemittel grundsätzlich jede chemische Verbindung eingesetzt werden kann, die die zum Kneten und/oder Verformen gewünschte Viskosität der zu verknetenden und/oder verformenden Masse gewährleistet. Demgemäß können Bindemittel im Sinne der vorliegenden Erfindung sowohl Viskositätserhöhende als auch Viskositätserniedrigende Verbindungen sein.

Als unter anderem bevorzugte Bindemittel sind beispielsweise Aluminiumoxid oder Aluminiumoxid enthaltende Binder, wie sie beispielsweise in der WO 94/29408 beschrieben sind, Siliciumdioxid, wie es beispielsweise in der EP 0 592 050 A1 beschrieben ist, Mischungen ais Siliciumdioxid und Aluminiumoxid, wie sie beispielsweise in der WO 94/13584 beschrieben sind, Tonminerale, wie sie beispielsweise in der JP 03-037156 A beschrieben sind, beispielsweise Montmorillonit, Kaolin, Bentonit, Halloysit, Dickit, Nacrit und Anauxit, Alkoxysilane, wie sie beispielsweise in der EP 0 102 544 B1 beschrieben sind, beispielsweise Tetraalkoxysilane wie beispielsweise Tetramethoxysilan, Tetraethoxysilan, Tetrapropoxysilan, Tetrabutoxysilan, oder beispielsweise Trialkoxysilane wie beispielsweise Trimethoxysilan, Triethoxysilan, Tripropoxysilan, Tributoxysilan, Alkoxytitanate, beispielsweise Tetraalkoxytitanate wie beispielsweise Tetramethoxytitanat, Tetraethoxytitanat, Tetrapropoxytitanat, Tetrabutoxytitanat, oder beispielsweise Trialkoxytitanate wie beispielsweise Trimethoxytitanat, Triethoxytitanat, Tripropoxytitanat, Tributoxytitanat, Alkoxyzirkonate, beispielsweise Tetraalkoxyzirkonate wie beispielsweise Tetramethoxyzirkonat, Tetraethoxyzirkonat, Tetrapropoxyzirkonat, Tetrabutoxyzirkonat, oder beispielsweise Trialkoxyzirkonate wie beispielsweise Trimethoxyzirkonat, Triethoxyzirkonat, Tripropoxyzirkonat, Tributoxyzirkonat, Silikasole, amphiphile Substanzen und/oder Graphite zu nennen.

Als viskositätssteigernde Verbindung kann beispielsweise auch, gegebenenfalls zusätzlich zu den oben genannten Verbindungen, eine organische Verbindung und/oder ein hydrophiles Polymer wie beispielsweise Cellulose oder ein Cellulosederivat wie beispielsweise Methylcellulose und/oder ein Polyacrylat und/oder ein Polymethacrylat und/oder ein Polyvinylalkohol und/oder ein Polyvinylpyrrolidon und/oder ein Polyisobuten und/oder ein Polytetrahydrofuran und/oder ein Polyethylenoxid eingesetzt werden.

Als Anteigungsmittel kann unter anderem bevorzugt Wasser oder mindestens ein Alkohol wie beispielsweise ein Monoalkohol mit 1 bis 4 C-Atomen wie beispielsweise Methanol, Ethanol, n-Propanol, iso-Propanol, 1-Butanol, 2-Butanol, 2-Methyl-1-propanol oder 2-Methyl-2-propanol oder ein Gemisch aus Wasser und mindestens einem der genannten Alkohole oder ein mehrwertiger Alkohol wie beispielsweise ein Glykol, bevorzugt ein wassermischbarer mehrwertiger Alkohol, allein oder als Gemisch mit Wasser und/oder mindestens einem der genannten einwertigen Alkohole eingesetzt werden.

Weitere Additive, die zum Kneten und/oder Verformen eingesetzt werden können, sind unter anderem Amine oder Aminderivate wie beispielsweise Tetraalkylammonium-Verbindungen oder Aminoalkohole und Carbonat enthaltende Verbindungen wie etwa Calciumcarbonat. Solche weiteren Additive sind etwa in der EP 0 389 041 A1, der EP 0 200 260 A1 oder der WO 95/19222 beschrieben.

Die Reihenfolge der Additive wie Templatverbindung, Binder, Anteigungsmittel, viskositätssteigernde Substanz beim Verformen und Kneten ist grundsätzlich nicht kritisch.

Gemäß einer weiteren bevorzugten Ausführungsform wird der gemäß Kneten und/oder Verformen erhaltene Formkörper mindestens einer Trocknung unterzogen, die im Allgemeinen bei einer Temperatur im Bereich von 25 bis 500 °C, bevorzugt im Bereich von 50 bis 500 °C und besonders bevorzugt im Bereich von 100 bis 350 °C durchgeführt wird. Ebenso ist es möglich, im Vakuum oder unter Schutzgasatmosphäre oder durch Sprühtrocknung zu trocknen.

Gemäß einer besonders bevorzugten Ausführungsform wird im Rahmen dieses Trocknungsvorgangs mindestens eine der als Additive zugesetzten Verbindungen zumindest teilweise aus dem Formkörper entfernt.

Das erfindungsgemäße metallorganische Gerüstmaterial enthält Poren, insbesondere Mikro- und/oder Mesoporen. Mikroporen sind definiert als solche mit einem Durchmesser von 2 nm oder kleiner und Mesoporen sind definiert durch einen Durchmesser im Bereich von 2 bis 50 nm (Pure & Appl. Chem. 57 (1985) 603-619). Die Anwesenheit von Mikro- und/oder Mesoporen kann mit Hilfe von Sorptionsmessungen überprüft werden, wobei diese Messungen die Aufnahmekapazität der metallorganischen Gerüstmaterialien für Stickstoff bei 77 Kelvin (nach Langmuir) gemäß DIN 66131 und/oder DIN 66134 bestimmt.

Vorzugsweise beträgt die spezifische Oberfläche - berechnet nach dem Langmuir-Modell (DIN 66131, 66134) für das erfindungsgemäße metallorganische Gerüstmaterial in Pulverform mindestens 1000 m²/g, mehr bevorzugt mindestens 1200 m²/g, mehr bevorzugt mindestens 1400 m²/g, weiter mehr bevorzugt mindestens 1600 m²/g, weiter mehr bevorzugt mindestens 1650 m²/g.

Formkörper aus dem erfindungsgemäßen metallorganischen Gerüstmaterial können eine niedrigere spezifische Oberfläche besitzen; vorzugsweise jedoch mindestens 500 m²/g, mehr bevorzugt mindestens 600 m²/g, weiter mehr bevorzugt mindestens 700 m²/g, insbesondere mindestens 800 m²/g.

Das erfindungsgemäße poröse metallorganische Gerüstmaterial kann dadurch erhalten werden, dass ein Reaktionsgemisch umgesetzt wird, das mindestens eine Aluminium verbindung enthält und der Terephthalsäure oder einem Salz davon in Gegenwart des Hilfsstoffes. Die Reaktion findet in einem organischen Lösemittel bei einer vorgegebenen Temperatur und einem vorgegebenen Druck statt.

Als organische Komponente des erfindungsgemäßen porösen metallorganischen Gerüstmaterials dient Terephthalsäure, die mit einer Metallverbindung umgesetzt werden kann. Ebenso ist es möglich, Derivate der Terephthalsäure einzusetzen. So ist es zum Beispiel denkbar, dass die Terephthalsäure in Form ihres Salzes eingesetzt wird. Das Salz, bei dem die Terephthalsäure als vollständig oder teilweise deprotoniertes Anion vorliegt, kann ein beliebig geeignetes Kation aufweisen.

Solche Kationen können beispielsweise ein- oder zweiwertige, vorzugsweise einwertige Metallionen sein. Beispiele hierfür sind insbesondere Natrium- und Kaliumsalze. Ebenso sind Kationen von Ammoniumverbindungen einsetzbar. Hierbei sind insbesondere Ammonium selbst sowie Alkylammoniumkationen zu nennen.

Die Aluminiumverbindung kann durch anodische Oxidation des Aluminiums erzeugt werden. In einem solchen Fall wird das erfindungsgemäße poröse metallorganische Gerüstmaterial auf zumindest teilweise elektrochemischem Wege hergestellt. Verfahren zur elektrochemischen Herstellung von porösen metallorganischen Gerüstmaterialien sind in WO-A 2005/049892 beschrieben. Auch die Erzeugung der Metallverbindung für das erfindungsgemäße poröse metallorganische Gerüstmaterial kann auf diesem Wege durchgeführt werden.

Bei der elektrochemischen Herstellung des erfindungsgemäßen porösen metallorganischen Gerüstmaterials ist bevorzugt, dass die kathodische Wiederabscheidung des Metallions durch mindestens eine der folgenden Maßnahmen zumindest teilweise verhindert wird:
(i) Verwendung eines Elektrolyten, der die kathodische Bildung von Wasserstoff begünstigt;
(ii) Zusatz mindestens einer Verbindung, die zu einer kathodischen Depolarisation führt;
(iii) Einsatz einer Kathode mit einer geeigneten Wasserstoffüberspannung.

Das Verfahren kann in einer ungeteilten Elektrolysezelle durchgeführt werden. Speziell geeignete Zellen sind Spaltzellen oder Plattenstapelzellen. Diese können bipolar geschaltet sein. Als Reaktionsmedium eignet sich beispielsweise Methanol, Ethanol, Dimethylformamid, Diethylformamid oder ein Gemisch aus zwei oder mehr dieser Lösemittel.

In dem Reaktionsgemisch kann/können weiterhin ein Leitsalz oder mehrere Leitsalze vorhanden sein. Hierbei kann das Leitsalz als Kationkomponente ein quartäres Ammonium und als Anionkomponente ein Alkoxysulfat aufweisen. Der Gesamtfeststoffgehalt sollte im Bereich von größer oder gleich 0,5 Gew.-% liegen.

Die Umsetzung in dem erfindungsgemäßen Verfahren zur Herstellung des erfindungsgemäßen metallorganischen Gerüstmaterials kann auch auf klassischem Wege erfolgen. Hierbei ist die Aluminiumverbindung typischerweise ein Aluminiumsalz.

Das Aluminiumsalz kann in Form eines Alkoholats, Acetonats, Halegonids, Sulfits, als Salz einer organischen oder anorganischen, Sauerstoff enthaltenden Säure oder einer Mischung davon vorliegen.

Ein Alkoholat ist beispielsweise ein Methanolat, Ethanolat, n-Propanolat, i-Propanolat, n-Butanolat, i-Butanolat, t-Butanolat oder Phenolat.

Ein Acetonat ist beispielsweise Acetylacetonat.

Ein Halogenid ist beispielsweise Chlorid, Bromid oder Iodid.

Eine organische, Sauerstoff enthaltende Säure ist beispielsweise Ameisensäure, Essigsäure, Propionsäure oder andere Alkylmonocarbonsäuren.

Eine anorganische, Sauerstoff enthaltende Säure ist beispielsweise Schwefelsäure, schweflige Säure, Phosphorsäure oder Salpetersäure.

Die Aluminiumverbindung kann gegebenenfalls Hydratwasser aufweisen, was bevorzugt ist. Besonders bevorzugt sind als Aluminiumverbindung die Hydrate des Chlorids, Nitrats sowie Sulfats.

Das erfindungsgemäße Verfahren findet in Gegenwart des anspruchs gemäßen Hilfsstoffes statt. Dies ist erforderlich, damit die thermodynamisch ungünstigere erfindungsgemäße Struktur für die erfindungsgemäßen metallorganischen Gerüstmaterialien erhalten wird anstelle des im Stand der Technik bekannten Strukturaufbaus "MIL-53".

Die Hilfsstoffe sind 3,5-Dihydroxybenzoesäure, 2,5-Dihydroxybenzoesäure, Schleimsäure, Glutamin, 4-Sulformoylbenzoesäure, 2-Mercaptonicotinsäure, Calconcarbonsäure oder Sulfasalazin.

Besonders bevorzugt sind Calconcarbonsäure und Sulfasalazin, insbesondere Sulfasalazin.

Die Umsetzung in dem erfindungsgemäßen Verfahren zur Herstellung des erfindungsgemäßen porösen metallorganischen Gerüstmaterials erfolgt zumindest in Gegenwart eines organischen Lösemittels. Hierbei können Solvothermalbedingungen eingesetzt werden.

Unter dem Begriff "thermal" ist im Rahmen der vorliegenden Erfindung ein Herstellverfahren zu verstehen, bei dem die Umsetzung zum erfindungsgemäßen porösen metallorganischen Gerüstmaterial in einem Druckbehälter derart durchgeführt wird, dass dieser während der Umsetzung verschlossen ist und erhöhte Temperatur angelegt wird, so dass aufgrund des Dampfdruckes von vorhandenem Lösemittel sich ein Druck innerhalb des Reaktionsmediums im Druckbehälter aufbaut.

Vorzugsweise erfolgt die Umsetzung nicht in Wasser enthaltendem Medium und ebenso nicht unter Solvothermalbedingungen.

Die Umsetzung in dem erfindungsgemäßen Verfahren erfolgt demzufolge vorzugsweise in Gegenwart eines nicht-wässrigen Lösemittels.

Die Umsetzung erfolgt vorzugsweise bei einem Druck von 1 bar bis 16 bar (absolut), vorzugsweise 1 bis 3 bar (absolut). Weiter bevorzugt beträgt der Druck jedoch höchstens 1230 mbar (absolut). Insbesondere bevorzugt findet die Umsetzung bei Atmosphärendruck statt. Hierbei kann es jedoch apparativ bedingt zu leichten Über- oder Unterdrücken kommen. Daher ist im Rahmen der vorliegenden Erfindung unter dem Begriff "Atmosphärendruck" derjenige Druckbereich zu verstehen, der sich aus dem tatsächlichen vorliegenden Atmosphärendruck ± 150 mbar ergibt.

Die Umsetzung findet vorzugsweise in einem Temperaturbereich von 100 °C bis 200 °C statt. Vorzugsweise liegt die Temperatur im Bereich von 110 °C bis 170 °C. Weiterhin bevorzugt liegt die Temperatur in einem Bereich von 120 °C bis 150 °C.

Das Reaktionsgemisch kann weiterhin eine Base aufweisen. Dies dient insbesondere dazu, dass die Dicarbonsäure leicht löslich ist. Durch die Verwendung eines organischen Lösemittels ist es häufig nicht erforderlich, eine solche Base einzusetzen. Nichts desto trotz kann das Lösemittel für das erfindungsgemäße Verfahren derart gewählt werden, dass dieses als solches basisch reagiert, was jedoch nicht zwingend für die Durchführung des erfindungsgemäßen Verfahrens sein muss.

Ebenso kann eine Base eingesetzt werden. Bevorzugt ist jedoch, dass keine zusätzliche Base eingesetzt wird.

Es ist weiterhin vorteilhaft, dass die Umsetzung unter Rühren stattfinden kann, was auch bei einem Scale-up vorteilhaft ist.

Das (nicht-wässrige) organische Lösemittel ist vorzugsweise ein C₁₋₆-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogeniertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann bzw. können. Beispiele hierfür sind Chloroform, Dichlormethan, Tetrachlormethan, Dichlorethan, Hexan, Heptan, Oktan sowie Gemische davon.

Bevorzugte Lösemittel sind DMF, DEF, DMAc und NMP. Besonders bevorzugt ist DMF.

Der Begriff "nicht-wässrig" bezieht sich vorzugsweise auf ein Lösemittel, das einen Höchstwassergehalt von 10 Gew.%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Vorzugsweise beträgt der Höchstwassergehalt während der Umsetzung 10 Gew.-%, mehr bevorzugt 5 Gew.-% und weiterhin mehr bevorzugt 1 Gew.-%.

Der Begriff "Lösemittel" betrifft reine Lösemittel sowie Gemische von unterschiedlichen Lösemitteln.

Weiterhin bevorzugt schließt sich an den Verfahrensschritt der Umsetzung der mindestens einen Metallverbindung mit der mindestens einen zweizähnigen organischen Verbindung ein Calcinierungsschritt an. Die hierbei eingestellte Temperatur beträgt typischerweise mehr als 250 °C, bevorzugt 300 bis 400 °C.

Aufgrund des Calcinierungsschrittes kann die in den Poren befindliche mindestens zweizähnige organische Verbindung entfernt werden.

Ergänzend oder alternativ hierzu kann die Entfernung der mindestens zweizähnigen organischen Verbindung (Ligand) aus den Poren des porösen metallorganischen Gerüstmaterials durch die Behandlung des gebildeten Gerüstmaterials mit einem nicht-wässrigen Lösemittel erfolgen. Hierbei wird in einer Art "Extraktionsverfahren" der Ligand entfernt und gegebenenfalls im Gerüstmaterial durch ein Lösemittelmolekül ersetzt.

Die Behandlung erfolgt vorzugsweise mindestens 30 Minuten und kann typischerweise bis zu 7 Tagen durchgeführt werden. Dies kann bei Raumtemperatur oder erhöhter Temperatur geschehen. Vorzugsweise erfolgt dies unter erhöhter Temperatur, beispielsweise bei mindestens 40 °C, bevorzugt 60 °C. Weiterhin bevorzugt erfolgt die Extraktion bei der Siedetemperatur des eingesetzten Lösemittels statt (unter Rückfluss).

Die Behandlung kann in einem einfachen Kessel durch Aufschlämmen und Rühren des Gerüstmaterials erfolgen. Es können auch Extraktionsapparaturen wie Soxhlet-Apparaturen, insbesondere technische Extraktionsapparaturen, eingesetzt werden.

Als geeignete Lösemittel können die oben genannten verwendet werden, also beispielsweise C₁₋₆-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon oder Mischungen davon.

Bevorzugt sind Methanol, Ethanol, Propanol, Aceton, MEK und Mischungen davon.

Ein ganz besonders bevorzugtes Extraktionslösemittel ist Methanol.

Das verwendete Lösemittel zur Extraktion kann gleich oder verschieden zu demjenigen für die Umsetzung der mindestens einen Metallverbindung mit der mindestens einen mindestens zweizähnigen organischen Verbindung sein. Es ist bei der "Extraktion" nicht unbedingt erforderlich aber bevorzugt, dass das Lösemittel wasserfrei ist.

Ein weiterer Gegenstand der vorliegenden Erfindung ist die Verwendung eines erfindungsgemäßen porösen metallorganischen Gerüstmaterials zur Aufnahme mindestens eines Stoffes, zu dessen Speicherung, Abtrennung, kontrollierten Abgabe, chemischen Umsetzung oder als Träger.

Vorzugsweise handelt es sich bei dem mindestens einen Stoff um ein Gas oder Gasgemisch. Auch Flüssigkeiten sind möglich.

Darüber hinaus eignet sich das erfindungsgemäße poröse metallorganische Gerüstmaterial zur Herstellung der entsprechenden Metalloxide. Solche Umwandlungen sind in der internationalen Anmeldung mit der Anmeldenummer PCT/EP2007/053571 beschrieben. Dabei erfolgt die Erhitzung des metallorganischen Gerüstmaterials über dessen vollständige Zersetzungstemperatur, so dass aus dem metallorganischen Gerüstmaterial, welches das mindestens eine Metallion enthält, das dem mindestens einen Metallion entsprechende Metalloxid oder entsprechende Metalloxidgemische erhalten werden. Hierbei kann vorteilhafterweise die spezifische Oberfläche des Gerüstmaterials auch bei dem Metalloxid(gemisch) wieder gefunden werden.

Daher ist ein weiterer Gegenstand der vorliegenden Erfindung die Verwendung eines metallorganischen Gerüstmaterials zur Herstellung eines entsprechenden Metalloxides. Im Rahmen der vorliegenden Erfindung wird vereinfachend der Begriff "Metalloxid" verwendet, auch wenn es sich um ein Metalloxidgemisch handelt.

Die erfindungsgemäßen Verwendungen des metallorganischen Gerüstmaterials gelten für Formkörper diese enthaltend entsprechend.

Verfahren zur Speicherung mit Hilfe von metallorganischen Gerüstmaterialien im Allgemeinen sind in WO-A 2005/003622, WO-A 2003/064030, WO-A 2005/049484, WO-A 2006/089908 sowie DE-A 10 2005 012 087 beschrieben. Die dort beschriebenen Verfahren können auch für das erfindungsgemäße metallorganische Gerüstmaterial eingesetzt werden. Bevorzugte Gase zur Speicherung sind Methan oder Wasserstoff.

Verfahren zur Trennung beziehungsweise Reinigung mit Hilfe von metallorganischen Gerüstmaterialien im Allgemeinen sind in der EP-A 1 674 555, DE-A 10 2005 000938 und in der deutschen Patentanmeldung mit der Anmeldenummer DE-A 10 2005 022 844 beschrieben. Die dort beschriebenen Verfahren können auch für das erfindungsgemäße metallorganische Gerüstmaterial eingesetzt werden. Ein bevorzugt abzutrennendes Gas ist Kohlendioxid, insbesondere aus einem weiterhin Kohlenmonoxid enthaltenden Gasgemisch.

Sofern das erfindungsgemäße poröse metallorganische Gerüstmaterial zur Speicherung eingesetzt wird, erfolgt dies vorzugsweise in einem Temperaturbereich von -200 °C bis +80 °C. Mehr bevorzugt ist ein Temperaturbereich von -40 °C bis +80 °C. Ein bevorzugter Druckbereich liegt bei 20 bar bis 1000 bar (absolut), insbesondere 100 bar bis 400 bar.

Im Rahmen der vorliegenden Erfindung werden vereinfachend die Begriffe "Gas" und "Flüssigkeit" verwendet, wobei hier jedoch ebenso Gasgemische sowie Flüssigkeitsgemische beziehungsweise flüssige Lösungen unter dem Begriff "Gas" beziehungsweise "Flüssigkeit" zu verstehen sind.

Bevorzugte Gase sind Wasserstoff, Erdgas, Stadtgas, Kohlenwasserstoffe, insbesondere Methan, Ethan, Ethen, Acetylen, Propan, n-Butan sowie i-Butan, Kohlenmonoxid, Kohlendioxid, Stickoxide, Sauerstoff, Schwefeloxide, Halogene, Halogenide Kohlenwasserstoffe, NF₃, SF₆, Ammoniak, Borane, Phosphane, Schwefelwasserstoff, Amine, Formaldehyd, Edelgase, insbesondere Helium, Neon, Argon, Krypton sowie Xenon.

Besonders bevorzugt handelt es sich bei dem Gas um Kohlendioxid, das aus einem Kohlendioxid enthaltenden Gasgemisch abgetrennt wird. Bevorzugt weist dabei das Gasgemisch neben Kohlendioxid wenigstens H₂, CH₄ oder Kohlenmonoxid auf. Insbesondere weist dabei das Gasgemisch neben Kohlendioxid Kohlenmonoxid auf. Ganz besonders bevorzugt sind Gemische, die wenigstens 10 und höchsten 45 Vol.% Kohlendioxid und wenigstens 30 und höchstens 90 Vol.-% Kohlenmonoxid enthalten.

Eine bevorzugte Ausführungsform ist die Druckwechseladsorption mit mehreren parallelen Adsorberreaktoren, wobei die Adsorbensschüttung ganz oder teilweise aus dem erfindungsgemäßen Material besteht. Die Adsorptionsphase findet für die CO₂/CO-Trennung bevorzugt bei einem CO₂-Partialdruck von 0,6 bis 3 bar und Temperatur von wenigstens 20, jedoch höchstens 70°C statt. Zur Desorption des adsorbierten Kohlendioxids wird der Gesamtdruck in dem betreffenden Adsorberreaktor üblicherweise abgesenkt auf Werte zwischen 100 mbar und 1 bar.

Weiterhin bevorzugt ist die Verwendung des erfindungsgemäßen Gerüstmaterials zum Speichern eines Gases bei einem Mindestdruck von 100 bar (absolut). Mehr bevorzugt beträgt der Mindestdruck 200 bar (absolut), insbesondere 300 bar (absolut). Hierbei handelt es sich besonders bevorzugt bei dem Gas um Wasserstoff oder Methan.

Bei dem mindestens einen Stoff kann es sich jedoch auch um eine Flüssigkeit handeln. Beispiele für eine solche Flüssigkeit sind Desinfektionsmittel, anorganische oder organische Lösemittel, Treibstoffe - insbesondere Benzin oder Diesel -, Hydraulik-, Kühler-, Bremsflüssigkeit oder ein Öl, insbesondere Maschinenöl. Weiterhin kann es sich bei der Flüssigkeit um halogenierte aliphatische oder aromatische, cyclische oder acyclische Kohlenwasserstoffe oder Mischungen davon handeln. Insbesondere kann die Flüssigkeit Aceton, Acetonitril, Anilin, Anisol, Benzol, Benzonitril, Brombenzol, Butanol, tert.-Butanol, Chinolin, Chlorbenzol, Chloroform, Cyclohexan, Diethylenglykol, Diethylether, Dimethylacetamid, Dimethylformamid, Dimethylsulfoxid, Dioxan, Eisessig, Essigsäureanhydrid, Essigsäureethylester, Ethanol, Ethylencarbonat, Ethylendichlorid, Ethylenglykol, Ethylenglykoldimethylether, Formamid, Hexan, Isopropanol, Methanol, Methoxypropanol, 3-Methyl-1-butanol, Methylenchlorid, Methylethylketon, N-Methylformamid, N-Methylpyrrolidon, Nitrobenzol, Nitromethan, Piperidin, Propanol, Propylencarbonat, Pyrridin, Schwefelkohlenstoff, Sulfolan, Tetrachlorethen, Tetrachlorkohlenstoff, Tetrahydrofuran, Toluol, 1,1,1-Trichlorethan, Trichlorethylen, Triethylamin, Triethylenglykol, Triglyme, Wasser oder Mischungen hiervon handeln.

Weiterhin kann der mindestens eine Stoff ein Geruchsstoff sein.

Vorzugsweise handelt es sich bei dem Geruchsstoff um eine flüchtige organische oder anorganische Verbindung, die mindestens eines der Elemente Stickstoff, Phosphor, Sauerstoff, Schwefel, Fluor, Chlor, Brom oder lod enthält oder ein ungesättigter oder aromatischer Kohlenwasserstoff oder ein gesättigter oder ungesättigter Aldehyd oder ein Keton ist. Mehr bevorzugte Elemente sind Stickstoff, Sauerstoff, Phosphor, Schwefel, Chlor, Brom; insbesondere bevorzugt sind Stickstoff, Sauerstoff, Phosphor und Schwefel.

Insbesondere handelt es sich bei dem Geruchsstoff um Ammoniak, Schwefelwasserstoff, Schwefeloxide, Stickoxide, Ozon, cyclische oder acyclische Amine, Thiole, Thioether sowie Aldehyde, Ketone, Ester, Ether, Säuren oder Alkohole. Besonders bevorzugt sind Ammoniak, Schwefelwasserstoff, organische Säuren (bevorzugt Essigsäure, Propionsäure, Buttersäure, Isobuttersäure, Valeriansäure, Isovaleriansäure, Capronsäure, Heptylsäure, Laurinsäure, Pelargonsäure) sowie cyclische oder acyclische Kohlenwasserstoffe, die Stickstoff oder Schwefel enthalten sowie gesättigte oder ungesättigte Aldehyde, wie Hexanal, Heptanal, Oktanal, Nonanal, Decanal, Octenal oder Nonenal und insbesondere flüchtige Aldehyde wie Butyraldehyd, Propionaldehyd, Acetaldehyd und Formaldehyd und weiterhin Treibstoffe wie Benzin, Diesel (Inhaltsstoffe).

Bei den Geruchsstoffen kann es sich auch um Riechstoffe, die beispielsweise zur Herstellung von Parfümen verwendet werden, handeln. Beispielhaft seien als Riechstoffe oder Öle, die solche Riechstoffe freisetzen zu nennen: ätherische Öle, Basilikumöl, Geranienöl, Minzöl, Canangabaumöl, Kardamomöl, Lavendelöl, Pfefferminzöl, Muskatöl, Kamillenöl, Eukalyptusöl, Rosmarinöl, Zitronenöl, Limettenöl, Orangenöl, Bergamottenöl, Muskateller Salbeiöl, Korianderöl, Zypressenöl, 1,1-Dimethoxy-2-pherylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid, Ethyl-2-methylpentanoat, 1-(2,6,6-Trimethyl-1,3-cyclohexadien-1-yl)-2-buten-1-on, Ethylvanillin, 2,6-Dimethyl-2-octenol, 3,7-Dimethyl-2-octenol, tert-Butylcyclohexylacetat, Anisylacetate, Allylcyclohexyloxyacetat, Ethyllinalool, Eugenol, Cumarin, Ethylacetacetat, 4-Phenyl-2,4,6-trimethyl-1,3-dioxan, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonate, 2,6-Dimethyl-5-hepten-1-al, 4-(Tricyclo[5.2.1.0]decylidene)-8-butanal, 5-(2,2,3-Trimethyl-3-cyclopentenyl)-3-methylpentan-2-ol, p-tert-Butylalpha-methylhydrozimtaldehyd, Ethyl[5.2.1.0]tricyclodecancarboxylat, Geraniol, Citronellol, Citral, Linalool, Linalylacetat, Jonone, Phenylethanol oder Mischungen hiervon.

Im Rahmen der vorliegenden Erfindung weist ein flüchtiger Geruchsstoff vorzugsweise einen Siedepunkt oder Siedepunktsbereich von weniger als 300 °C auf. Mehr bevorzugt ist der Geruchsstoff eine leicht flüchtige Verbindung oder Gemisch. Insbesondere bevorzugt weist der Geruchsstoff einen Siedepunkt oder Siedebereich von weniger als 250 °C, mehr bevorzugt weniger als 230 °C, insbesondere bevorzugt weniger als 200 °C auf.

Bevorzugt sind ebenfalls Geruchsstoffe, die eine hohe Flüchtigkeit aufweisen. Als Maß für die Flüchtigkeit kann der Dampfdruck herangezogen werden. Im Rahmen der vorliegenden Erfindung weist ein flüchtiger Geruchsstoff vorzugsweise einen Dampfdruck von mehr als 0,001 kPa (20 °C) auf. Mehr bevorzugt ist der Geruchsstoff eine leicht flüchtige Verbindung oder Gemisch. Insbesondere bevorzugt weist der Geruchsstoff einen Dampfdruck von mehr als 0,01 kPa (20 °C) auf, mehr bevorzugt einen Dampfdruck von mehr als 0,05 kPa (20 °C) auf. Besonders bevorzugt weisen die Geruchsstoffe einen Dampfdruck von mehr als 0,1 kPa (20 °C) auf.

Beispiele, bei der eine chemische Umsetzung in Gegenwart des erfindungsgemäßen metallorganischen Gerüstmaterials stattfinden kann, stellen die Alkoxylierung von Monoolen sowie Polyolen dar. Die Durchführung solcher Alkoxylierungen sind WO-A 03/035717 sowie WO-A 2005/03069 beschrieben. Ebenso kann das erfindungsgemäße poröse metallorganische Gerüstmaterial zur Epoxidierung sowie Herstellung von Polyalkylencarbonaten und Wasserstoffperoxid eingesetzt werden. Solche Reaktionen sind in WO-A 03/101975, WO-A 2004/037895 sowie US-A 2004/081611 beschrieben.

Besonders bevorzugt sind katalytische Umsetzungen.

Darüber hinaus kann das erfindungsgemäße metallorganische Gerüstmaterial als Träger, insbesondere als Träger eines Katalysators, dienen.

### Beispiele

### Beispiel 1 Herstellung eines AI-Terephthalsäure-MOFs mit Sulfasalazin als Hilfsstoff

4,88 g AlCl₃*6H₂O, 5 g Terephthalsäure und 2,99 g Sulfasalazin wird in 300 ml DMF in einem Glaskolben suspendiert. Das Gemisch wird unter Rühren auf 130°C aufgeheizt und 18,5 h unter diesen Bedingungen gehalten. Das angefallene Produkt wird abfiltriert und mit 3 x 50 ml DMF und 4 x 50 ml Methanol gewaschen. Anschließend wird das Produkt in eine Papierhülse überführt und in einer Extraktionsapparatur 120 h mit siedendem Methanol extrahiert. Abschließend wird das Produkt in einem Vakuumtrockenschrank 24 h bei 100°C getrocknet. Die Ausbeute beträgt 4,12 g.

Das XRD (Diffraktogramm der Röntgenbeugung) ist in Fig. 2 dargestellt. Es werden Reflexe bei 2Θ = ca. 5°, ca. 10° und ca. 15° gefunden, die in dem herkömmlichen Al-Terephthalat-MOF (MIL-53) nicht beobachtet werden. Es handelt sich also um eine neuartige Struktur. Die Oberfläche beträgt 1679 m²/g. Dies ist ebenfalls höher als die bekannten Oberflächenwerte für MIL-53 (bis zu 1500 m²/g). Laut Elementaranalyse enthält das Produkt 11,6 wt% Al, 44,2 wt% Kohlenstoff 2,8 wt% Wasserstoff, 0,7 wt% N₂ und lediglich 0,15 wt% Schwefel, das als Verunreinigung von dem Hilfsstoff stammt.

### Beispiel 2 Herstellung eines Al-Terephthalsäure-MOFs mit Calconcarbonsäure als Hilfsstoff

4,88 g AlCl₃*6H₂O, 5 g Terephthalsäure und 3,3 g Calconcarbonsäure werden in 300 ml DMF in einem Glaskolben suspendiert. Das Gemisch wird unter Rühren auf 130°C aufgeheizt und 18 h unter diesen Bedingungen gehalten. Das angefallene Produkt wird abfiltirert, mit 3 x 50 ml DMF und 4 x 50 ml Methanol gewaschen. Anschließend wird das Produkt in eine Papierhülse überführt und in einer Extraktionsapparatur 120 h mit siedendem Methanol extrahiert. Abschließend wird das Produkt in einem Vakuumtrockenschrank 72 h bei 110°C getrocknet. Die Ausbeute betrug 4,34 g.
Das Produkt weist dieselben Reflexe im Röntgendiffraktogramm (XRD) wie Beispiel 1 auf. Die Oberfläche beträgt 1594 m²/g. Laut Elementaranalyse enthält das Produkt 10,0 wt% Al, 46,1 wt% Kohlenstoff 3,7 wt% Wasserstoff, 3,2 wt% N₂ und lediglich 0,07 wt% Schwefel.

### Beispiel 3 Adsorption von CO₂ und CO

An jeweils ca. 200 mg Probe werden mit dem kommerziell erhältlichen Gerät ASAP 2420 (Fa. Micromeritics) Adsorptionsisothermen mit CO und CO₂ zwischen 0 und 1.2 bar gemessen. Die Messtemperatur beträgt 40°C. Die Proben werden vor der Messung jeweils mehrere Stunden unter Vakuumbedingungen (Turbomolekularpumpe) bei 200°C vorbehandelt.

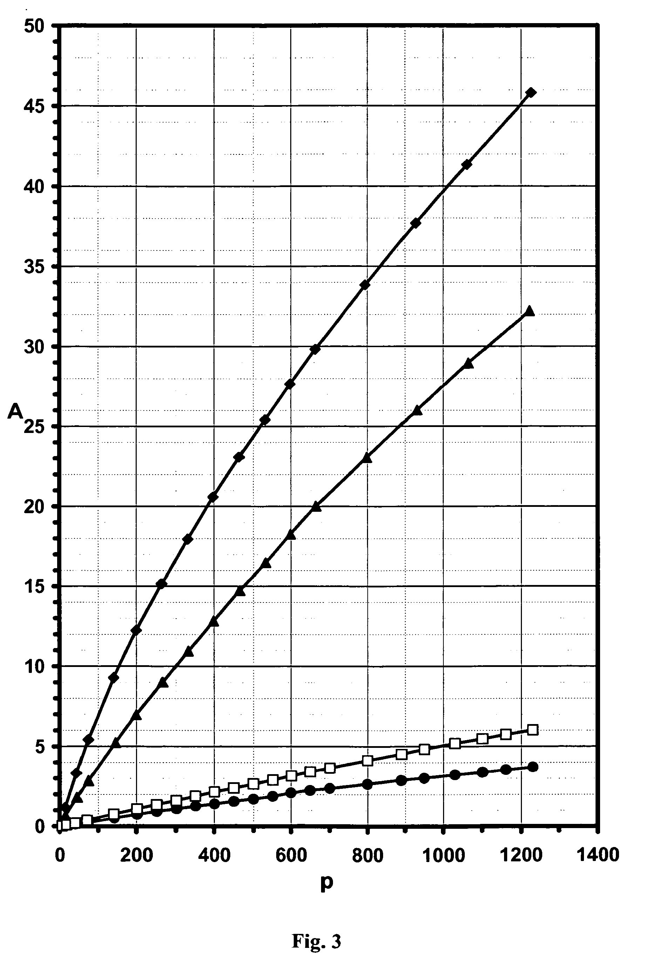

Fig. 3 zeigt die jeweiligen Isothermen im Vergleich zu Al-Terephthalsäure aus dem Stand der Technik. Hierbei ist die Gasaufnahme A (in Nml/g) als Funktion des Drucks p (in mbar) dargestellt.

Vermessen werden eine Probe gemäß Beispiel 1 (mit Dreiecken gekennzeichnete Kurve: CO₂-Adsorption; mit Kreisen gekennzeichnete Kurve: CO-Adsorption in Fig. 3) sowie ein Al-Terepthalsäure-MOF mit "MIL-53" Struktur aus dem Stand der Technik, hergestellt nach Beispiel 12 der WO-A 2007/023134 (mit ausgefüllten Quadraten gekennzeichnete Kurve: CO₂-Adsorption, mit nicht ausgefüllten Quadraten gekennzeichnete Kurve: CO-Adsorption in Fig. 3).

Der insgesamt flachere Verlauf der Adsorptionsisothermen auf dem erfindungsgemäßen Material korreliert mit einer geringeren Adsorptionsenergie, so dass weniger Energie für eine Regenerierung des Materials nach Adsorption (z. B. in der Desorptionsphase bei einer Druckwechseladsorption) erforderlich ist. Zudem ist die relative Differenz zwischen der CO₂- und der CO-Kapazität hilfreich, da dies eine höhere Selektivität und damit höhere erzielbare Reinheiten für eine Trennung von CO₂ von CO bedeutet.

## Patentansprüche

1. Poröses metallorganisches Gerüstmaterial, enthaltend eine an ein Metallion koordinativ gebundene, zweizähnige organische Verbindung, wobei das Metallion ausgewählt ist aus der Gruppe der Metalle bestehend aus Al, Fe und Cr und wobei die zweizähnige organische Verbindung sich von einer Dicarbonsäure ableitet, **dadurch gekennzeichnet, dass** das Gerüstmaterial eine Kristallstruktur aufweist, deren Projektion entlang [001] ein Muster aufweist, bei dem jede Seite eines Sechsecks durch ein Dreieck begrenzt ist, wobei das Metall Al und die zweizähnige organische Verbindung Terephthalsäure ist.

2. Gerüstmaterial nach Anspruch 1, **dadurch gekennzeichnet, dass** das Röntgendiffraktogramm des Gerüstmaterials einen Hauptreflex im Bereich von 4° < 2Θ < 6° aufweist.

3. Formkörper enthaltend ein poröses metallorganische Gerüstmaterial nach Anspruch 1 oder 2.

4. Verfahren zur Herstellung eines porösen metallorganischen Gerüstmaterials nach Anspruch 1 oder 2, den Schritt enthaltend
- Umsetzung eines Reaktionsgemisches enthaltend mindestens eine Metallverbindung, wobei das Metall Al ist und Terephthalsäure oder einem Salz davon in Gegenwart eines Hilfsstoffes, der mindestens eine deprotonierbare Gruppe aufweist, in einem organischen Lösemittel bei einer vorgegebenen Temperatur und einem vorgegebenen Druck, wobei der Hilfsstoff 3,5-Dihydroxybenzoesäure, 2,5-Dihydroxybenzoesäure, Schleimsäure, Glutamin, 4-Sulfamoylbenzoesäure, 2-Mercaptonicotinsäure, Calconcarbonsäure oder Sulfasalazin ist.

5. Verwendung eines metallorganischen Gerüstmaterials nach Anspruch 1 oder 2 oder eines Formkörpers nach Anspruch 3 zur Aufnahme mindestens eines Stoffes zu dessen Speicherung, Abtrennung, kontrollierten Abgabe, chemischen Umsetzung; als Träger oder zur Herstellung eines entsprechenden Metalloxides.

## Claims

1. A porous metal organic framework comprising a bidentate organic compound coordinated to a metal ion selected from the group of metals consisting of Al, Fe and Cr, with the bidentate organic compound being derived from a dicarboxylic acid, wherein the framework has a crystal structure whose projection along [001] has a pattern in which each side of a hexagon is bounded by a triangle, and the metal is Al and the bidentate organic compound is terephthalic acid.

2. The framework according to claim 1, wherein the X-ray diffraction pattern of the framework has a main reflection in the range 4° < 2Θ < 6°.

3. A shaped body comprising a porous metal organic framework according to claim 1 or 2.

4. A process for preparing a porous metal organic framework according to claim 1 or 2, which comprises the step
- reaction of a reaction mixture comprising at least one compound of a metal, where the metal is Al, and terephthalic acid or a salt thereof in the presence of an auxiliary having at least one deprotonatable group in an organic solvent at a predetermined temperature and a predetermined pressure, wherein the auxiliary is 3,5-dihydroxybenzoic acid, 2,5-dihydroxybenzoic acid, mucic acid, glutamine, 4-sulfamoylbenzoic acid, 2-mercaptonicotinic acid, calconcarboxylic acid or sulfasalazine.

5. The use of a metal organic framework according to claim 1 or 2 or a shaped body according to claim 3 for the uptake of at least one substance for the purposes of its storage, separation, controlled release, chemical reaction; as support or for preparing a corresponding metal oxide.

## Revendications

1. Matériau de squelette métallo-organique poreux, contenant un composé organique bidentate relié coordinativement à un ion métallique, l'ion métallique étant choisi dans le groupe des métaux constitué par Al, Fe et Cr, et le composé organique bidentate dérivant d'un acide dicarboxylique, **caractérisé en ce que** le matériau de squelette présente une structure cristalline dont la projection le long de [001] présente un motif selon lequel chaque côté d'un hexagone est délimité par un triangle, le métal étant Al et le composé organique bidentate étant l'acide téréphtalique.

2. Matériau de squelette selon la revendication 1, **caractérisé en ce que** le diffractogramme de rayons X du matériau de squelette présente une réflexion principale dans la plage de 4° < 2θ < 6°.

3. Corps moulé contenant un matériau de squelette métallo-organique poreux selon la revendication 1 ou 2.

4. Procédé de fabrication d'un matériau de squelette métallo-organique poreux selon la revendication 1 ou 2, contenant l'étape suivante :
- la mise en réaction d'un mélange réactionnel contenant au moins un composé métallique, le métal étant Al, et de l'acide téréphtalique ou un de ses sels en présence d'un adjuvant, qui comprend au moins un groupe déprotonable, dans un solvant organique à une température prédéterminée et à une pression prédéterminée, l'adjuvant étant l'acide 3,5-dihydroxybenzoïque, l'acide 2,5-dihydroxybenzoïque, l'acide mucique, la glutamine, l'acide 4-sulfamoylbenzoïque, l'acide 2-mercaptonicotinique, l'acide calconecarboxylique ou la sulfasalazine.

5. Utilisation d'un matériau de squelette métallo-organique selon la revendication 1 ou 2 ou d'un corps moulé selon la revendication 3 pour l'absorption d'au moins une substance pour son stockage, sa séparation, sa libération contrôlée, sa transformation chimique ; en tant que support ou pour la fabrication d'un oxyde métallique correspondant.
